(19) Europäisches Patentamt
European Patent Office
Office européen des brevets

(11) EP 0 798 296 B1

(12) **EUROPÄISCHE PATENTSCHRIFT**

(45) Veröffentlichungstag und Bekanntmachung des Hinweises auf die Patenterteilung:
**10.12.2003 Patentblatt 2003/50**

(51) Int Cl.[7]: **C07D 213/81**, C07D 239/42, C07D 241/24, C07D 253/06, B01J 31/24

(21) Anmeldenummer: **97103981.3**

(22) Anmeldetag: **10.03.1997**

(54) **Verfahren zur Herstellung von Arylamiden Heteroaromatischer Carbonsäuren**

Process of preparation of Arylamides Heteroaromatic Carboxylic Acids

Procédé pour la préparation des arylamides des acides carboxyliques hétéroaromatiques

(84) Benannte Vertragsstaaten:
**AT BE CH DE DK ES FI FR GB GR IE IT LI LU MC NL PT SE**

(30) Priorität: **21.03.1996 CH 73596**

(43) Veröffentlichungstag der Anmeldung:
**01.10.1997 Patentblatt 1997/40**

(73) Patentinhaber: **Lonza AG**
**3930 Visp (CH)**

(72) Erfinder:
- **Roduit, Jean-Paul, Dr.**
  **3979 Grône (Kanton Wallis) (CH)**
- **Kalbermatten, Georges**
  **3938 Ausserberg (Kanton Wallis) (CH)**

(74) Vertreter: **Winter, Brandl, Fürniss, Hübner, Röss, Kaiser, Polte Partnerschaft**
**Patent- und Rechtsanwaltskanzlei**
**Alois-Steinecker-Strasse 22**
**85354 Freising (DE)**

(56) Entgegenhaltungen:
**EP-A- 0 053 011**
**EP-A- 0 282 266**
**EP-A- 0 282 658**
**EP-A- 0 447 004**
**EP-A- 0 627 422**
**EP-A- 0 646 590**

- **A. SCHOENBERG ET AL.: "Palladium-catalyzed Amidation of Aryl, Heterocyclic, and Vinylic Halides" JOURNAL OF ORGANIC CHEMISTRY, Bd. 39, Nr. 23, 1974, Seiten 3327-3331, XP000673766 COLUMBUS OHIO**
- **RYO TAKEUCHI ET AL.: "Palladium-catalyzed carbonylation of N-heteroaromatic chloride" JOURNAL OF MOLECULAR CATALYSIS, Bd. 66, Nr. 3, 1991, Seiten 277-288, XP002037142 THE NETHERLANDS**
- **YEHOSHUA BEN-DAVID ET AL.: "Chelate-Assisted, Pd-Catalyzed Efficient Carbonylation of Aryl Chlorides" JOURNAL OF AMERICAN CHEMICAL SOCIETY, Bd. 111, Nr. 23, 1989, Seiten 8742-8744, XP002037143 COLUMBUS OHIO**
- **PATENT ABSTRACTS OF JAPAN vol. 11, no. 369 (C-461) [2816], 2. Dezember 1987 & JP 62 142161 A (MITSUI TOATSU CHEM. INC.), 25. Juni 1987**
- **LORENZO TESTAFERI ET AL.: "The reaction of some halogenated pyridines with methoxide and methanethiolate ions in Dimethylformamide" TETRAHEDRON, Bd. 41, Nr. 7, 1985, Seiten 1373-1384, XP002081893 great britain**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 798 296 B1

## Beschreibung

Die vorliegende Erfindung betrifft ein Verfahren zur Herstellung von Arylamiden heteroaromatischer Carbonsäuren durch Umsetzung von heteroaromatischen Halogenverbindungen mit Kohlenmonoxid und aromatischen Aminen in Gegenwart eines Katalysators und einer Base.

Die erfindungsgemäss herstellbaren Amide besitzen die allgemeine Formel

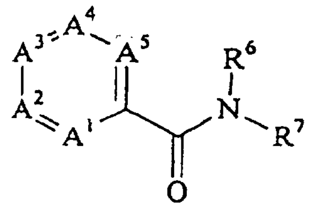

I.

Hierin bedeuten:

$A^1$ Stickstoff oder $CR^1$,
$A^2$ Stickstoff oder $CR^2$,
$A^3$ Stickstoff oder $CR^3$,
$A^4$ Stickstoff oder $CR^4$,
und $A^5$ Stickstoff oder $CR^5$,
mit der Massgabe, dass wenigstens eines der Ringglieder $A^1$ bis $A^5$ Stickstoff ist und nicht zwei Stickstoffatome unmittelbar miteinander verbunden sind;
$R^1$ bis $R^5$, soweit vorhanden, unabhängig voneinander Wasserstoff, $C_{1-4}$-Alkyl oder Aryl, wobei einer der Substituenten $R^1$ bis $R^5$ auch eine Gruppe der Formel -OR sein kann, in welcher R ein gegebenenfalls substituierter aromatischer oder heteroaromatischer Rest ist;
$R^6$ Wasserstoff oder $C_{1-4}$-Alkyl
und $R^7$ einen gegebenenfalls substituierten aromatischen oder heteroaromatischen Rest. Insbesondere gehören hierzu die Arylamide der Pyridin-, Pyrimidin-, Pyrazin- und 1,3,5-Triazincarbonsäuren.
Zahlreiche Verbindungen dieser Struktur, insbesondere solche, bei denen einer der Substituenten $R^1$ bis $R^5$ eine Aryloxygruppe (-OR) in Nachbarschaft zu einem Ringstickstoffatom ist, sind wichtige Herbizide (WO-A 94/27974, EP-A 0 053 011, EP-A 0 447 004).

Die Synthese dieser bekannten Verbindungen geht üblicherweise von den entsprechenden Carbonsäuren oder Carbonsäurederivaten (Säurechloride, Ester, Nitrile) aus. Diese sind jedoch oft schwer zugänglich und deshalb teuer.
Aufgabe der vorliegenden Erfindung war daher, ein alternatives Verfahren zur Verfügung zu stellen. welches von leichter zugänglichen Edukten ausgeht.
Erfindungsgemäss wird diese Aufgabe durch das Verfahren nach Patentanspruch 1 gelöst.
Es wurde gefunden, dass Halogenverbindungen der allgemeinen Formel

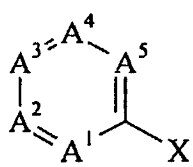

II,

worin $A^1$ bis $A^5$ die oben genannten Bedeutungen haben und X Chlor, Brom oder Iod ist, mit Kohlenmonoxid und einem primären oder sekundären Amin der allgemeinen Formel

$$R^6\text{-NH-}R^7 \qquad \text{III,}$$

worin $R^6$ und $R^7$ die oben genannten Bedeutungen haben, in Gegenwart einer Base in guter bis fast quantitativer Ausbeute direkt zu den gewünschten Produkten (I) reagieren, wenn als Katalysator ein Komplex von Palladium mit einem Diphosphin der allgemeinen Formel

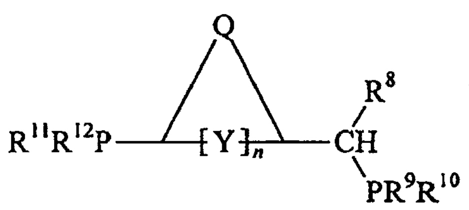

IV,

anwesend ist. Es bedeuten hierin:

$R^8$ Wasserstoff oder $C_{1-4}$-Alkyl,
$R^9$ bis $R^{12}$ unabhängig voneinander sekundäres oder tertiäres $C_{3-6}$-Alkyl, $C_{5-7}$-Cycloalkyl oder gegebenenfalls substituiertes Phenyl,
Y $CH_o$, $NH_p$ oder Sauerstoff,
*n* 0 oder 1,
*o* 1 oder 2
*p* 0 oder 1
und Q ein verbrückendes organisches Radikal, das zusammen mit den beiden benachbarten Kohlenstoffatomen und, falls vorhanden ($n$ = 1), mit Y einen gegebenenfalls substituierten fünf- oder sechsgliedrigen gesättigten oder aromatischen carbo- oder heterocyclischen Ring bildet, der als aromatischer Ring gegebenenfalls mit einem Übergangsmetall komplexiert sein kann.

Unter $C_{1-4}$-Alkyl sind hier und im folgenden alle linearen oder verzweigten primären, sekundären oder tertiären Alkylgruppen mit bis zu 4 Kohlenstoffatomen zu verstehen. Unter aromatischen oder heteroaromatischen Resten sind hier und im folgenden insbesondere mono- oder polycyclische Systeme wie beispielsweise Phenyl, Naphthyl, Biphenylyl, Anthracenyl, Furyl, Pyrrolyl, Pyrazolyl, Thiophenyl, Pyridyl, Indolyl oder Chinolinyl zu verstehen. Diese können einen oder mehrere gleiche oder verschiedene Substituenten tragen, beispielsweise Halogene wie Chlor, Brom oder Fluor, niedrige Alkylgruppen wie Methyl, halogenierte Alkylgruppen wie Trifluormethyl, niedrige Alkoxygruppen wie Methoxy, oder niedrige Alkylthio- (Alkansulfanyl-) oder Alkansulfonylgruppen wie Methylthio oder Ethansulfonyl.
Unter mit Übergangsmetallen komplexierten aromatischen Ringen sind insbesondere $\eta^5$-Cyclopentadienyl-Ringe und $\eta^6$-Benzolringe in Sandwich- und Halbsandwich-Komplexen wie Metallocenen oder verwandten Verbindungen zu verstehen, beispielsweise in Ferrocen oder Benzolchromtricarbonyl.
Die als Ausgangsmaterial dienenden Halogenverbindungen (II) sind bekannte Verbindungen oder können analog zu bekannten Verbindungen hergestellt werden. Zahlreiche derartige Verbindungen sind beispielsweise in US-A 4 254 125 und in EP-A 0 001 187 offenbart.
Vorzugsweise eignet sich das erfindungsgemässe Verfahren zur Herstellung solcher Amide (I), in denen $A^2$ Stickstoff ist und mit den übrigen Ringgliedern einen Pyridinring bildet.
Besonders bevorzugt sind dabei jene Amide (I), in denen $R^1$ eine Gruppe der Formel -OR ist, wobei R die oben genannte Bedeutung hat.
Ebenfalls bevorzugt sind solche Amide (I), in denen $A^1$ Stickstoff ist und mit den übrigen Ringgliedern einen Pyridinring bildet,
solche, in denen $A^1$ und $A^5$ Stickstoff sind und mit den übrigen Ringgliedem einen Pyrimidinring bilden,
solche, in denen $A^1$ und $A^4$ Stickstoff sind und mit den übrigen Ringgliedern einen Pyrazinring bilden,
sowie solche, in denen $A^1$, $A^3$ und $A^5$ Stickstoff sind und mit den übrigen Ringgliedern einen 1,3,5-Triazinring bilden.
Unter den vier letztgenannten Klassen sind wiederum diejenigen Amide besonders bevorzugt, in welchen $R^2$ eine Gruppe der Formel -OR ist, wobei R die oben genannte Bedeutung hat.
Unter den Amiden (I), bei welchen einer der Substituenten $R^1$ bis $R^5$ eine Gruppe der Formel -OR ist, sind diejenigen bevorzugt, in denen R eine gegebenenfalls substituierte Phenylgruppe ist. Dies gilt insbesondere für die vorstehend genannten Amide mit Pyridin-, Pyrimidin-, Pyrazin- oder 1,3,5-Triazinring, in denen $R^1$ oder $R^2$ eine Gruppe der Formel -OR ist.
Ebenfalls bevorzugt sind solche Amide, in denen $R^6$ Wasserstoff und $R^7$ eine gegebenenfalls substituierte Phenylgruppe ist.

Als Halogenverbindungen (II) sind die Chlorverbindungen (X = Cl) bevorzugt.

Als Diphosphine (IV) werden vorzugsweise solche eingesetzt, in denen *n* = 0 ist und Q zusammen mit den beiden benachbarten Kohlenstoffatomen einen fünfgliedrigen Ring bildet, der Teil eines Ferrocensystems ist. Diese Verbindungen können durch die allgemeine Formel

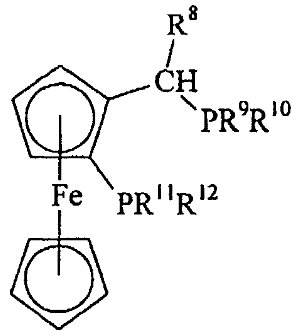

IVa,

worin $R^8$ bis $R^{12}$ die oben genannten Bedeutungen haben, dargestellt werden. Besonders bevorzugt sind dabei solche Diphosphine, bei denen $R^8$ Wasserstoff oder Methyl ist. Diese Verbindungen sind chiral und wurden (insbesondere wenn $R^8 \neq H$) als reine Stereoisomere beispielsweise für asymmetrische Hydrierungen eingesetzt (siehe z. B. EP-A 0 564 406, EP-A 0 612 758). Da bei dem erfindungsgemässen Verfahren kein Chiralitätselement neu gebildet wird, können diese Diphosphine hier auch als Racemate oder sonstige Stereoisomerengemische eingesetzt werden. Ganz besonders bevorzugt sind Diphosphine (IVa), in denen $R^9 = R^{10}$ und $R^{11} = R^{12}$ ist und diese Substituenten ausgewählt sind aus der Gruppe bestehend aus Isopropyl, *tert*-Butyl, Cyclohexyl und gegebenenfalls substituiertem Phenyl.

Ebenfalls bevorzugt sind solche Diphosphine (IV), in denen *n* = 0 ist und Q zusammen mit den beiden benachbarten Kohlenstoffatomen einen Benzol-, Pyridin-, Pyrrol- oder Furanring bildet. Beispielhaft ist hier das Tricarbonyl-$\eta^6$-{1-(diphenylphosphino)-2-[(1-(diphenylphosphino)ethyl]benzol}chrom(0) zu nennen (*J. Organometall. Chem*. **1995**, *503*, 143-148).
Desgleichen sind solche Diphosphine bevorzugt, in denen *n* = 1 ist, Y eine Methylengruppe ist und Y zusammen mit Q und den beiden benachbarten Kohlenstoffatomen einen Pyrrolidinring bildet, der gegebenenfalls weitere Substituenten trägt. Hierzu zählt beispielsweise das (2*S*,4*S*)-1-*tert*-Butoxycarbonyl-4-diphenylphosphino-2-(diphenylphosphinomethyl)pyrrolidin (BPPM) (*J. Org. Chem*. **1980,** *45,* 4680).

Der katalytisch wirksame Palladium-Diphosphin-Komplex wird vorteilhaft *in situ* gebildet, indem Palladium in fein verteilter elementarer Form (z. B. Palladium auf Aktivkohle), ein $Pd_{(II)}$ Salz (z. B. das Chlorid oder das Acetat), oder ein geeigneter $Pd_{(II)}$-Komplex (z. B. Dichloro-bis(triphenylphosphin)palladium$_{(II)}$) mit dem Diphosphin zur Reaktion gebracht wird. Das Palladium wird vorzugsweise in einer Menge von 0,02 bis 0,2 Mol-% $Pd_{(II)}$ oder 0,5 bis 2 Mol-% Pd(0) (als Pd/C), jeweils bezogen auf die Halogenverbindung (II), eingesetzt. Das Diphosphin wird vorteilhaft im Überschuss (bezogen auf Pd) eingesetzt, vorzugsweise in einer Menge von 0,2 bis 5 Mol-%, ebenfalls bezogen auf die Halogenverbindung (II).

Als Lösungsmittel können sowohl relativ unpolare, wie beispielsweise Toluol, Xylol oder Methylcyclohexan, als auch polare wie beispielsweise Acetonitril, Tetrahydrofuran, *N,N*-Dimethylacetamid oder Butylacetat eingesetzt werden.

Als Base wird vorzugsweise eine relativ schwache Base eingesetzt. Diese braucht in dem verwendeten Lösungsmittel nicht löslich zu sein. Gut geeignet sind beispielsweise Carbonate wie Natriumcarbonat oder Kaliumcarbonat, Acetate wie Natriumacetat, oder sekundäre oder tertiäre Phosphate wie Dikaliumhydrogenphosphat oder Trikaliumphosphat. Mit Natriumcarbonat oder Natriumacetat wurden ganz besonders gute Ergebnisse erzielt.

Die Reaktionstemperatur liegt vorzugsweise bei 80 bis 250 °C.

Der Kohlenmonoxiddruck liegt vorzugsweise bei 1 bis 50 bar.

Die folgenden Beispiele verdeutlichen die Durchführung des erfindungsgemässen Verfahrens.

**Beispiel 1**

**2-Chlor-6-[3-(trifluormethyl)phenoxy]pyridin**

In 420 ml *N,N*-Dimethylacetamid wurden 17,45 g (690 mmol) Natriumhydrid (95%) suspendiert. Bei 15 °C wurden innerhalb von 2 h 106,7 g (658 mmol) 3-(Trifluormethyl)-phenol zugetropft. Die so erhaltene Phenolatlösung wurde innerhalb von 2,5 h unter Stickstoff zu einer auf 90 °C erwärmten Lösung von 162,4 g (1,097 mol) 2,6-Dichlorpyridin in 330 ml *N,N*-Dimethylacetamid getropft. Nach weiteren 3 h Reaktionszeit wurde das Gemisch auf Raumtemperatur abgekühlt, das ausgefallene Natriumchlorid abfiltriert und das Filtrat eingeengt. Der Rückstand wurde mit Toluol und 0,1 N Salzsäure aufgenommen, die organische Phase mit gesättigter Natriumchloridlösung gewaschen und eingeengt. Der ölige Rückstand (ca. 200 g) wurde im Vakuum destilliert.

Ausbeute: 151,5 g (84%) farbloses Öl, Gehalt (GC) 99,8%

$n_D^{20}$ = 1,5267
MS; *m/z*: 273/275; 238; 39

$^1$H NMR ($CDCl_3$): δ = 6,84 (d, *J* = 7,8 Hz, 1H); 7,07 (d, *J* = 7,8 Hz, 1H); 7,35 (m, 1H); 7,42 (m, 1H); 7,45-7,52 (m, 2H); 7,65 (t, *J* = 7,8 Hz, 1H).

$^{13}$C NMR ($CDCl_3$): δ = 109,88 (CH); 118,16 (CH); 119,24 (CH); 121,67 (CH); 123,74 ($CF_3$); 124,50 (CH); 130,24 (CH); 132,21 ($CCF_3$); 141,77 (CH); 149,12 (C); 153,89 (C); 162,28 (C).

**Beispiel 2**

**3-Chlor-2-[3-(trifluormethyl)phenoxy]pyridin**

Unter Stickstoff wurden 7,68 g Natriumhydrid-Dispersion (ca. 50 % in Mineralöl) mit Pentan gewaschen, dann wurden 100 ml *N,N*-Dimethylformamid zugegeben. Bei Raumtemperatur wurden innerhalb von 30 min 21,92 g (135 mmol) 3-(Trifluormethyl)phenol zugetropft. Die so erhaltene Phenolatlösung wurde innerhalb von 2 h unter Stickstoff zu einer auf 120 °C erhitzten Lösung von 20,1 g (136 mmol) 2,3-Dichlorpyridin in 80 ml *N,N*-Dimethylformamid getropft. Nach 3 h Reaktionsdauer wurde das Gemisch auf Raumtemperatur abgekühlt, das ausgefallene Natriumchlorid abfiltriert und das Filtrat eingeengt. Der Rückstand wurde mit Toluol und 0,1 N Salzsäure extrahiert, die organische Phase mit gesättigter Natriumchloridlösung gewaschen und eingeengt. Der ölige Rückstand wurde im Vakuum destilliert.

Ausbeute: 24,75 g (67%) farbloses Öl, Gehalt (GC) 99,7%

Sdp.$_{18\ mbar}$ = 145-148 °C
$n_D^{20}$ = 1,5282

MS; *m/z*: 273/275
$^1$H NMR ($CDCl_3$) : δ = 6,99 (m, 1H); 7,36 (d, 1H); 7,45-7,53 (m, 3H); 7,77 (d, 1H); 8,02 (d, 1H).
$^{13}$C NMR ($CDCl_3$) : δ = 118,66 (CH); 119,44 (C); 119,98 (CH); 121,75 (CH); 123,78 ($CF_3$); 124,94 (CH); 130,13 (CH); 132,16 ($CCF_3$); 139,65 (CH); 145,20 (CH); 153,88 (C); 158,51 (C).

**Beispiel 3**

***N*-(4-Fluorphenyl)-6-[3-(trifluormethyl)phenoxy]pyridin-2-carboxamid**

In einem Autoklaven wurden bei Raumtemperatur 6,84 g (25 mmol) 2-Chlor-6-[3-(trifluormethyl)phenoxy]pyridin (Gehalt 99,5%, hergestellt nach Beispiel 1), 4,17 g (37,5 mmol) 4-Fluoranilin, 2,92 g (27,5 mmol) Natriumcarbonat, 17,5 mg (25 μmol) Dichloro-bis(triphenylphosphin)palladium$_{(II)}$ und 0,31 g (0,75 mmol) (±)-1-[2-(Diphenylphosphino)-ferrocenyl]ethyl-diphenylphosphin (IVa, $R^8$ = Methyl, $R^9$ = $R^{10}$ = $R^{11}$ = $R^{12}$ = Phenyl, hergestellt nach A. Togni et al., *Inorg. Chim. Acta* **1994**, *222*, 213-224) in 25 ml Xylol vorgelegt. Der Autoklav wurde mit Inertgas gespült, dann wurden 5 bar Kohlenmonoxid aufgepresst und auf 200 °C aufgeheizt. Der CO-Druck wurde auf 16 bar erhöht und das Gemisch 21 h bei 200 °C gerührt. Nach dem Abkühlen auf Raumtemperatur und der Druckentlastung wurde das Reaktionsgemisch mit je 50 ml Xylol und Wasser versetzt und filtriert. Die wässrige Phase wurde mit 25 ml Xylol extrahiert und die vereinigten organischen Phasen mit 30 ml Wasser gewaschen. Mittels GC wurde die Zusammensetzung der gelösten Produkte bestimmt. Gefunden wurden 97,8% Titelverbindung (Amid), 2,2% Nebenprodukt (sekundäres Amin, entstanden durch direkte Substitution von Cl durch das Anilin). Nach dem Abdestillieren des Lösungsmittels wurde das Rohprodukt als gelber Feststoff erhalten.
Rohausbeute (HPLC-Analyse, mit Standard) : 89,9%
Zur Reinigung wurde das Rohprodukt aus Methylcyclohexan umkristallisiert.

Ausbeute: 6,3 g (67%) farblose Kristalle

| | |
|---|---|
| Schmp.: | 104-105 °C |
| MS; *mlz*: | 376 ($M^+$), 238 |
| $^1H$ NMR ($CDCl_3$): δ = | 6,99-7,04 (m, 2H); 7,17 (d, *J* = 8,4 Hz, 1H); 7,40 (m, 1H); 7,46-7,51 (m, 2H); 7,55-7,63 (m, 3H); 7,93 (t, *J* = 7,8 Hz, 1H); 8,03 (d, *J* = 7,8 Hz, 1H); 9,24 (br. m, 1H). |

**Beispiel 4**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle des (±)-1-[2-(Diphenylphosphino) ferrocenyl]ethyl-diphenylphosphins die gleiche molare Menge (±)-1-[2-(Diphenylphosphino)ferrocenyl]ethyl-di-*tert*-butylphosphin (IVa, $R^8$ = Methyl, $R^9$ = $R^{10}$ = *tert*-Butyl, $R^{11}$ = $R^{12}$ = Phenyl) eingesetzt. Der CO-Druck betrug 19 bar. Mittels GC wurde die Zusammensetzung der gelösten Produkte in der Xylol-Phase bestimmt. Gefunden wurden 97,2% Titelverbindung (Amid), 2,8% Nebenprodukt (sekundäres Amin).

**Beispiel 5**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle des (±)-1-[2-(Diphenylphosphino) ferrocenyl]ethyl-diphenylphosphins die gleiche molare Menge (±)-1-[2-(Diphenylphosphino)ferrocenyl]ethyl-diisopropylphosphin (IVa, $R^8$ = Methyl, $R^9$ = $R^{10}$ = Isopropyl, $R^{11}$ = $R^{12}$ = Phenyl) eingesetzt. Der CO-Druck betrug 19 bar. Mittels GC wurde die Zusammensetzung der gelösten Produkte in der Xylol-Phase bestimmt. Gefunden wurden 96,7% Titelverbindung (Amid), 3,3% Nebenprodukt (sekundäres Amin).

**Beispiel 6**

***N*-(4-Fluorphenyl)-6-[3-(trifluormethyl)phenoxy]pyridin-2-carboxamid**

Es wurde verfahren wie in Beispiel 3 beschrieben, jedoch wurde anstelle des (±)-1-[2-(Diphenylphosphino) ferrocenyl]ethyl-diphenylphosphins die gleiche molare Menge (±)-1-[2-(Diisopropylphosphino)ferrocenyl]ethyl-di-*tert*-butylphosphin (IVa, $R^8$ = Methyl, $R^9$ = $R^{10}$ = *tert*-Butyl, $R^{11}$ = $R^{12}$ = Isopropyl) eingesetzt. Der CO-Druck betrug 19 bar. Mittels GC wurde die Zusammensetzung der gelösten Produkte in der Xylol-Phase bestimmt. Gefunden wurden 98,9% Titelverbindung (Amid), 1,1% Nebenprodukt (sekundäres Amin).

**Beispiel 7**

***N*-(4-Fluorphenyl)-6-[3-(trifluormethyl)phenoxy]pyridin-2-carboxamid**

Es wurde verfahren wie in Beispiel 4, jedoch wurde anstelle von Natriumcarbonat die gleiche molare Menge Natriumacetat als Base eingesetzt. Der CO-Druck betrug 19 bar. Mittels GC wurde die Zusammensetzung der gelösten Produkte in der Xylol-Phase bestimmt. Gefunden wurden 99,7% Titelverbindung (Amid), 0,2% Edukt und <0,1 % Nebenprodukt (sekundäres Amin).

**Beispiel 8**

***N*-(4-Fluorphenyl)-6-[3-(trifluormethyl)phenoxy]pyridin-2-carboxamid**

Es wurde verfahren wie in Beispiel 4, jedoch wurde anstelle von Dichloro-bis(tri-phenylphosphin)palladium (II) die gleiche molare Menge Palladium(II)chlorid eingesetzt. Der CO-Druck betrug 19 bar. Mittels GC wurde die Zusammensetzung der gelösten Produkte in der Xylol-Phase bestimmt. Gefunden wurden 96,7% Titelverbindung (Amid) und 3,3% Nebenprodukt (sekundäres Amin).

**Beispiel 9**

***N*-(4-Fluorphenyl)-6-[3-(trifluormethyl)phenoxy]pyridin-2-carboxamid**

Es wurde verfahren wie in Beispiel 7, jedoch wurde anstelle von Dichloro-bis(triphenylphosphin)palladium$_{(II)}$ die gleiche molare Menge Palladium$_{(II)}$acetat eingesetzt. Der CO-Druck betrug 19 bar. Mittels GC wurde die Zusammensetzung der gelösten Produkte in der Xylol-Phase bestimmt. Gefunden wurden 99,0% Titelverbindung (Amid) und 0,8% Nebenprodukt (2-[3-(Trifluormethyl)phenoxy]pyridin, entstanden durch Hydrogenolyse des Chlorids).

**Beispiel 10**

***N*-(4-Fluorphenyl)-6-[3-(trifluormethyl)phenoxy]pyridin-2-carboxamid**

Es wurde verfahren wie im Beispiel 3, jedoch wurden anstelle des Ferrocenylphosphins 0,21 g (0,75 mmol) (2*S*,4*S*)-1-*tert*-Butoxycarbonyl-4-(diphenylphosphino)-2-(diphenylphosphinomethyl)pyrrolidin (Fluka) eingesetzt. Die Reaktionszeit betrug 20 h, der CO-Druck 17 bar. Mittels GC wurde die Zusammensetzung der gelösten Produkte in der Xylol-Phase bestimmt. Gefunden wurden 98,7% Titelverbindung (Amid) und 1,1% Nebenprodukt (sekundäres Amin).

**Beispiel 11**

***N*-(4-Fluorphenyl)-6-[3-([trifluormethyl)phenoxy]pyridin-2-carboxamid**

Es wurde verfahren wie in Beispiel 4, jedoch wurden nur 75 µmol (±)-1-[2-(Diphenylphosphino)ferrocenyl] ethyl-di-*tert*-butylphosphin eingesetzt. Der CO-Druck betrug 19 bar. Mittels GC wurde die Zusammensetzung der gelösten Produkte in der Xylol-Phase bestimmt. Gefunden wurden 88,8% Titelverbindung (Amid), 7,4% unumgesetztes Edukt und 3,3% Nebenprodukt (sekundäres Amin).

**Beispiel 12**

***N*-(4-Fluorphenyl)-6-[3-(trifluormethyl)phenoxy]pyridin-2-carboxamid**

Es wurde verfahren wie in Beispiel 4, jedoch wurden nur 27,5 mmol 4-Fluoranilin eingesetzt. Der CO-Druck betrug 19 bar. Mittels GC wurde die Zusammensetzung der gelösten Produkte in der Xylol-Phase bestimmt. Gefunden wurden 97,3% Titelverbindung (Amid) und 2,7% Nebenprodukt (sekundäres Amin).

**Beispiel 13**

***N*-(4-Fluorphenyl)-6-[3-(trifluormethyl)phenoxy]pyridin-2-carboxamid**

In einem Autoklaven wurden bei Raumtemperatur 6,84 g (25 mmol) 2-Chlor-6-[3-(trifluormethyl)phenoxy]pyridin (Gehalt 99,5%, hergestellt nach Beispiel 1), 3,33 g (30 mmol) 4-Fluoranilin, 2,92 g (27,5 mmol) Natriumcarbonat, 2,8 mg (12,5 µmol) Palladium$_{(II)}$-acetat und 68 mg (125 µmol) (±)-1-[2-(Diphenylphosphino)-ferrocenyl]ethyl-di-*tert*-butylphosphin (IVa, $R^8$ = Methyl, $R^9$ = $R^{10}$ = *tert*-Butyl, $R^{11}$ = $R^{12}$ = Phenyl) in 25 ml Acetonitril vorgelegt. Der Autoklav wurde mit Inertgas gespült, dann wurden 5 bar Kohlenmonoxid aufgepresst und auf 150 °C aufgeheizt, wobei sich der Druck wurde auf 7,6 bar erhöhte. Das Gemisch wurde 4 h bei 150 °C gerührt. Nach dem Abkühlen auf Raumtemperatur und der Druckentlastung wurde das Lösungsmittel abdestilliert und der Rückstand bei 80 °C mit 90 ml Methylcyclohexan aufgenommen. Die so erhaltene Suspension wurde filtriert und der Filterkuchen mit 10 ml warmem Methylcyclohexan nachgewaschen. Beim Abkühlen des Filtrats auf 5 °C kristallisierte das Produkt aus.

Ausbeute: 8,11 g (86,2%) leicht beiger Feststoff

Schmp.: 104,5-105,2 °C

**Beispiel 14**

***N*-(2,4-Difluorphenyl)-2-[3-(trifluormethyl)phenoxy]pyridin-3-carboxamid** (Diflufenicam)

Analog zu Beispiel 3 wurden 6,84 g (25 mmol) 3-Chlor-2-(3-trifluormethyl)phenoxypyridin (hergestellt nach Beispiel 2), 4,84 g (37,5 mmol) 2,4-Difluoranilin, 2,92 g (27,5 mmol) Natriumcarbonat, 17,5 mg (25 µmol) Dichloro-bis (triphenylphosphin)-palladium$_{(II)}$ und 0,31 g (0,75 mmol) (±)-1-[2-(Diphenylphosphino)-ferrocenyl]ethyl-di*tert*-butylphosphin in 25 ml Xylol unter 15 bar CO-Druck bei 150 °C 19 h umgesetzt. Der Umsatz war ca. 70%. Die Aufarbeitung wurde wie in Beispiel 3 durchgeführt. Es wurden 6 g Rohprodukt als gelber kristalliner Feststoff erhalten. Zur Reinigung wurde aus 50 ml Methylcyclohexan umkristallisiert.

| | |
|---|---|
| Ausbeute: | 3,25 g (33%) weisser Feststoff |
| Schmp.: | 157-159 °C |
| MS; *m*/*z*: | 394 ($M^+$), 266 (100%) |
| $^1$H NMR ($CDCl_3$): δ = | 6,89-6,96 (m, 2H); 7,26 (m, 1H); 7,46 (m, 1H); 7,54-7,63 (m, 3H); 8,28 (dd; H); 8,52 (m, 1H); 8,71 (dd, 1H); 9,97 (br. s, 1H). |

**Beispiel 15**

***N*-(4-Fluorphenyl)-6-[3-(trifluormethyl)phenoxy]pyrazin-2-carboxamid**

Analog zu Beispiel 3 wurden 25 mmol 2-Chlor-6-[3-(trifluormethyl)phenoxy]pyrazin (hergestellt nach US-A 4 254 125, Example 21), 27,5 mmol 4-Fluoranilin, 2,92 g (27,5 mmol) Natriumcarbonat, 17,5 mg (25 µmol) Dichloro-bis (triphenylphosphin)-palladium$_{(II)}$ und 0,31 g (0,75 mmol) (±)-1-[2-(Diphenylphosphino)-ferrocenyl]ethyl-di*tert*-butylphosphin in 25 ml Xylol unter 17 bar CO-Druck bei 120 °C 21 h umgesetzt. Mittels GC wurde die Zusammensetzung der gelösten Produkte in der Xylol-Phase bestimmt. Gefunden wurden 65,3% Titelverbindung (Amid) und 34,7% Nebenprodukt (sekundäres Amin). Das Amid wurde durch Säulenchromatographie isoliert und gereinigt.

| | |
|---|---|
| Schmp.: | 109-110 °C, farbloser Feststoff |
| $^1$H NMR ($CDCl_3$): δ = | 7,02-7,05 (m, 2H); 7,43 (m, 1H); 7,48-7,53 (m, 2H); 7,58-7,65 (m, 3H); 8,67 (s, 1H); 8,94 (br. s, 1H); 9,22 (s, 1H). |

**Vergleichsbeispiel 1**

Es wurde verfahren wie in Beispiel 3 beschrieben, jedoch wurde anstelle von (±)-1-[2-(Diphenylphosphino)-ferrocenyl]ethyl-diphenylphosphin die gleiche molare Menge Triphenylphosphin eingesetzt. Nach einer Reaktionsdauer von 15,5 h bei 15 bar CO-Druck wurde mittels GC die Zusammensetzung der gelösten Produkte in der Xylol-Phase bestimmt. Gefunden wurden nur 43,2% des gewünschten Produkts, aber 56,8% unumgesetztes Edukt.

**Vergleichsbeispiel 2**

Es wurde verfahren wie in Beispiel 3 beschrieben, jedoch wurde anstelle von (±)-1-[2-(Diphenylphosphino)-ferrocenyl]ethyl-diphenylphosphin die gleiche molare Menge Tri-*n*-butylphosphin eingesetzt. Nach einer Reaktionsdauer von 15 h bei 14 bar CO-Druck wurde mittels GC die Zusammensetzung der gelösten Produkte in der Xylol-Phase bestimmt. Gefunden wurden nur Spuren (0,4%) des gewünschten Produkts, aber 96,8% unumgesetztes Edukt.

Vergleichsbeispiel 3

Es wurde verfahren wie in Beispiel 3 beschrieben, jedoch wurde anstelle von (±)-1-[2-(Diphenylphosphino)-ferrocenyl]ethyl-diphenylphosphin die gleiche molare Menge 1,2-Bis-(diphenylphosphino)ethan eingesetzt. Nach einer Reaktionsdauer von 20,2 h bei 14,7 bar CO-Druck wurde mittels GC die Zusammensetzung der gelösten Produkte in der Xylol-Phase bestimmt. Gefunden wurden nur Spuren (2,2%) des gewünschten Produkts, aber 97,7% unumgesetztes Edukt.

## Patentansprüche

**1.** Verfahren zur Herstellung von Amiden der allgemeinen Formel

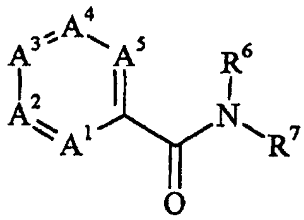

I,

worin
$A^1$ Stickstoff oder $CR^1$,
$A^2$ Stickstoff oder $CR^2$,
$A^3$ Stickstoff oder $CR^3$,
$A^4$ Stickstoff oder $CR^4$,
und $A^5$ Stickstoff oder $CR^5$ ist,
mit der Massgabe, dass wenigstens eines der Ringglieder $A^1$ bis $A^5$ Stickstoff ist und nicht zwei Stickstoffatome unmittelbar miteinander verbunden sind,
$R^1$ bis $R^5$, soweit vorhanden, unabhängig voneinander Wasserstoff, $C_{1-4}$-Alkyl, Phenyl, Naphthyl, Biphenylyl oder Anthracenyl sind, wobei einer der Substituenten $R^1$ bis $R^5$ auch eine Gruppe der Formel -OR sein kann, in welcher R gegebenenfalls mit Halogen, Methyl, Trifluormethyl, Methoxy, Methylthio und/oder Ethansulfonyl substituiertes Phenyl, Naphthyl, Biphenylyl, Anthracenyl, Furyl, Pyrrolyl, Pyrazolyl, Thiophenyl, Pyridyl, Indolyl- oder Chinolinyl ist,
$R^6$ Wasserstoff oder $C_{1-4}$-Alkyl
und $R^7$ gegebenenfalls wie oben substituiertes Phenyl, Naphthyl, Biphenylyl, Anthracenyl, Furyl, Pyrrolyl, Pyrazolyl, Thiophenyl, Pyridyl, Indolyl oder Chinolinyl ist, **dadurch gekennzeichnet, dass** eine Halogenverbindung der allgemeinen Formel

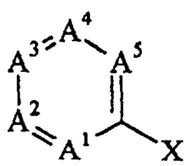

II,

worin $A^1$ bis $A^5$ die oben genannten Bedeutungen haben und X Chlor, Brom oder Iod ist, mit Kohlenmonoxid und einem Amin der allgemeinen Formel

$$R^6\text{-NH-}R^7 \qquad \text{III,}$$

worin $R^6$ und $R^7$ die oben genannten Bedeutungen haben, in Gegenwart eines Komplexes von Palladium mit einem Diphosphin der allgemeinen Formel

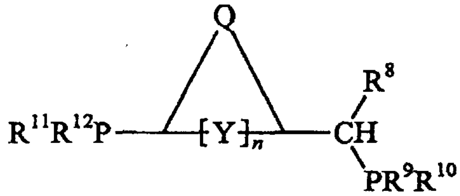

IV,

oder

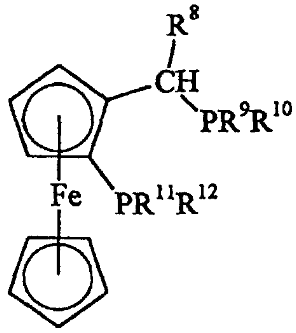

IVa,

worin $R^8$ Wasserstoff oder $C_{1-4}$-Alkyl ist,
$R^9$ bis $R^{12}$ unabhängig voneinander sekundäres oder tertiäres $C_{3-6}$-Alkyl, $C_{5-7}$-Cycloalkyl oder gegebenenfalls wie oben substituiertes Phenyl bedeuten,
Y $CH_2$ ist,
*n* 0 oder 1
und Q ein verbrückendes organisches Radikal ist, das wenn n = 0 zusammen mit den beiden benachbarten Kohlenstoffatomen einen Benzol-, Pyridin-, Pyrrol- oder Furanring bildet und wenn n = 1 zusammen mit den beiden benachbarten Kohlenstoffatomen einen gegebenenfalls wie oben substituierten Pyrrolidinring bildet,
wobei diese carbo- oder heterocyclischen Ringe als aromatische Ringe gegebenenfalls mit einem Übergangsmetall komplexiert sein können,
und einer Base zur Reaktion gebracht wird.

**2.** Verfahren nach Anspruch 1, **dadurch gekennzeichnet, dass** $A^2$ Stickstoff und Teil eines Pyridinringes ist.

**3.** Verfahren nach Anspruch 2, **dadurch gekennzeichnet, dass** $R^1$ eine Gruppe der Formel - OR ist, wobei R die in Anspruch 1 genannte Bedeutung hat.

**4.** Verfahren nach Anspruch 1, **dadurch gekennzeichnet, dass** $A^1$ Stickstoff und Teil eines Pyridinringes ist.

**5.** Verfahren nach Anspruch 1, **dadurch gekennzeichnet, dass** $A^1$ und $A^5$ Stickstoff und Teil eines Pyrimidinringes sind.

**6.** Verfahren nach Anspruch 1, **dadurch gekennzeichnet, dass** $A^1$ und $A^4$ Stickstoff und Teil eines Pyrazinringes sind.

**7.** Verfahren nach Anspruch 1, **dadurch gekennzeichnet, dass** $A^1$, $A^3$ und $A^5$ Stickstoff sind.

**8.** Verfahren nach Anspruch 4, 5, 6 oder 7, **dadurch gekennzeichnet, dass** $R^2$ eine Gruppe der Formel -OR ist, wobei R die in Anspruch 1 genannte Bedeutung hat.

**9.** Verfahren nach Anspruch 3 oder 8, **dadurch gekennzeichnet, dass** R eine gegebenenfalls substituierte Phenylgruppe ist.

**10.** Verfahren nach einem der Ansprüche 1 bis 9, **dadurch gekennzeichnet, dass** $R^6$ Wasserstoff und $R^7$ eine gegebenenfalls substituierte Phenylgruppe ist.

**11.** Verfahren nach einem der Ansprüche 1 bis 10, **dadurch gekennzeichnet, dass** X Chlor ist.

**12.** Verfahren nach einem der Ansprüche 1 bis 11, **dadurch gekennzeichnet, dass** als Diphosphin (IV) ein Ferrocen der allgemeinen Formel

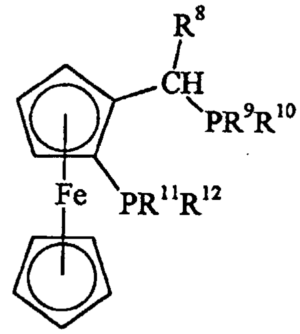

IVa,

worin $R^8$ Wasserstoff oder Methyl ist und $R^9$ bis $R^{12}$ die in Anspruch 1 genannten Bedeutungen haben, eingesetzt wird.

## Claims

**1.** Process for the production of amides of the general formula

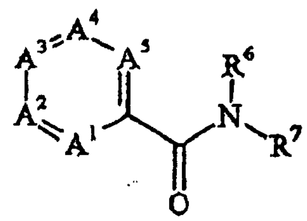

I,

wherein
$A^1$ is nitrogen or $CR^1$,
$A^2$ is nitrogen or $CR^2$,
$A^3$ is nitrogen or $CR^3$,
$A^4$ is nitrogen or $CR^4$,
and $A^5$ is nitrogen or $CR^5$,
with the proviso that at least one of the ring members $A^1$ to $A^5$ is nitrogen and two nitrogen atoms are not directly joined to one another,
$R^1$ to $R^5$, where present, independently of one another are hydrogen, $C_{1-4}$-alkyl, phenyl, naphthyl, biphenylyl or anthracenyl, wherein one of the substituents $R^1$ to $R^5$ may also be a group of the formula -OR in which R is phenyl, naphthyl, biphenylyl, anthracenyl, furyl, pyrrolyl, pyrazolyl, thiophenyl, pyridyl, indolyl or quinolyl, optionally substituted with halogen, methyl, trifluoromethyl, methoxy, methylthio and/or ethanesulfonyl,
$R^6$ is hydrogen or $C_{1-4}$-alkyl
and $R^7$ is phenyl, naphthyl, biphenylyl, anthracenyl, furyl, pyrrolyl, pyrazolyl, thiophenyl, pyridyl, indolyl or quinolyl optionally substituted as above, **characterised in that** a halogen compound of the general formula

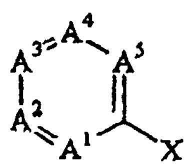

II,

wherein $A^1$ to $A^5$ have the meanings given above and X is chlorine, bromine or iodine, is reacted with carbon monoxide and an amine of the general formula

$$R^6\text{-NH-}R^7 \qquad III,$$

wherein $R^6$ and $R^7$ have the meanings given above, in the presence of a complex of palladium with a diphosphine of the general formula

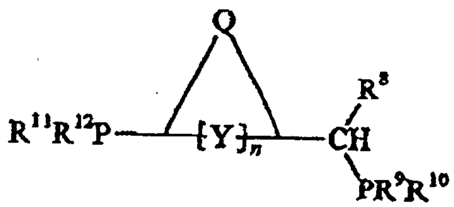

IV,

or

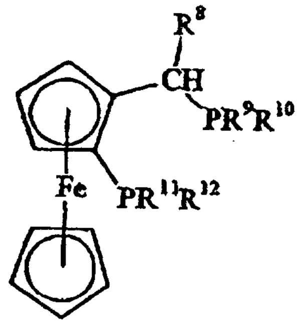

IVa,

wherein $R^8$ is hydrogen or $C_{1-4}$-alkyl,
$R^9$ to $R^{12}$ independently of one another denote secondary or tertiary $C_{3-6}$-alkyl, $C_{5-7}$-cycloalkyl, or phenyl optionally substituted as above,
Y is $CH_2$,
*n* is 0 or 1
and Q is a bridging organic radical which when n = 0 forms a benzene, pyridine, pyrrole or furan ring together with the two adjacent carbon atoms, and when n = 1 forms together with the two adjacent carbon atoms a pyrrolidine ring optionally substituted as above,
wherein these carbocyclic or heterocyclic rings as aromatic rings may optionally be complexed with a transition metal,
and with a base.

2. Process according to claim 1, **characterised in that** $A^2$ is nitrogen and is part of a pyridine ring.

3. Process according to claim 2, **characterised in that** $R^1$ is a group of the formula -OR where R has the meaning given in claim 1.

4. Process according to claim 1, **characterised in that** $A^1$ is nitrogen and is part of a pyridine ring.

5. Process according to claim 1, **characterised in that** $A^1$ and $A^5$ are nitrogen and are part of a pyrimidine ring.

6. Process according to claim 1, **characterised in that** $A^1$ and $A^4$ are nitrogen and are part of a pyrazine ring.

7. Process according to claim 1, **characterised in that** $A^1$, $A^3$ and $A^5$ are nitrogen.

8. Process according to claim 4, 5, 6 or 7, **characterised in that** $R^2$ is a group of the formula -OR where R has the

meaning given in claim 1.

**9.** Process according to claim 3 or 8, **characterised in that** R is an optionally substituted phenyl group.

**10.** Process according to one of claims 1 to 9, **characterised in that** $R^6$ is hydrogen and $R^7$ is an optionally substituted phenyl group.

**11.** Process according to one of claims 1 to 10, **characterised in that** X is chlorine.

**12.** Process according to one of claims 1 to 11, **characterised in that** as diphosphine (IV) there is used a ferrocene of the general formula

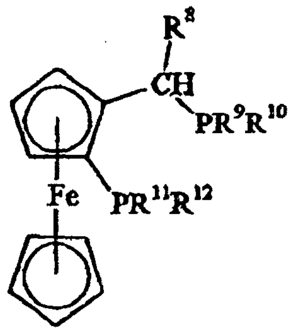

IVa,

wherein $R^8$ is hydrogen or methyl and $R^9$ to $R^{12}$ have the meanings given in claim 1.

**Revendications**

**1.** Procédé destiné à la production d'amides de formule générale

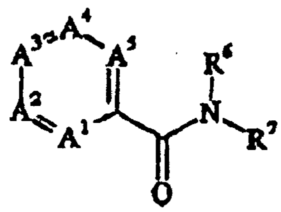

I,

dans laquelle
$A^1$ est un azote ou un $CR^1$ ;
$A^2$ est un azote ou un $CR^2$ ;
$A^3$ est un azote ou un $CR^3$ ;
$A^4$ est un azote ou un $CR^4$ ;
et $A^5$ est un azote ou un $CR^5$,
à la condition qu'au moins l'un des éléments du cycle $A^1$ à $A^5$ soit un azote et que deux atomes d'azote ne soient pas directement liés l'un à l'autre,
$R^1$ à $R^5$, dans la mesure où ils existent, sont indépendamment l'un de l'autre un hydrogène, un alkyle en $C_1$-$C_4$, un phényle, un naphtyle, un biphénylyle ou un anthracényle, un des substituants $R^1$ à $R^5$ pouvant également être un groupe de formule -OR, dans lequel R est un phényle, un naphtyle, un biphénylyle, un anthracényle, un furyle, un pyrrolyle, un pyrazolyle, un thiophényle, un pyridyle, un indolyle ou un quinolinyle, le cas échéant substitués par un halogène, un méthyle, un trifluorométhyle, un méthoxy, un méthylthio et / ou un éthane-sulfonyle,
$R^6$ est un hydrogène ou un alkyle en $C_{1-4}$ ;
et $R^7$ est un phényle, un naphtyle, un biphénylyle, un anthracényle, un furyle, un pyrrolyle, un pyrazolyle, un

thiophényle, un pyridyle, un indolyle ou un quinolinyle, le cas échéant substitués tel qu'indiqué ci-dessus, **caractérisé en ce qu'**un composé halogéné de formule générale

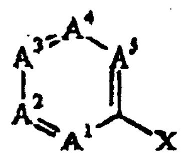
II,

dans laquelle $A^1$ à $A^5$ ont les significations citées ci-dessus et X est un chlore, un brome ou un iode, est porté à réaction avec du monoxyde de carbone et une amine de formule générale

$$R^6\text{-NH-}R^7 \qquad \text{III,}$$

dans laquelle $R^6$ et $R^7$ ont les significations citées ci-dessus, en présence d'un complexe de palladium avec une diphosphine de formule générale

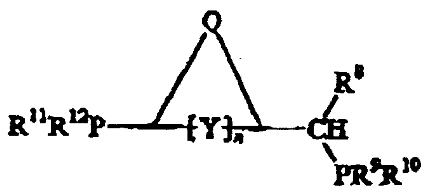
IV,

ou

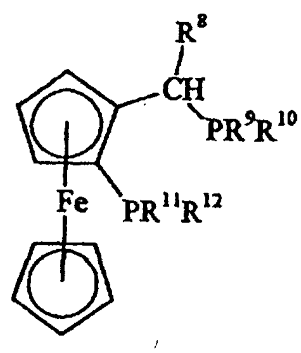
IVa,

où $R^8$ est un hydrogène ou un alkyle en $C_{1-4}$,
$R^9$ à $R^{12}$ représentent indépendamment l'un de l'autre un alkyle secondaire ou tertiaire en $C_{3-6}$, un cycloalkyle en $C_{5-7}$ ou un phényle le cas échéant substitué tel que mentionné ci-dessus,
Y est $CH_2$,
*n* est égal à 0 ou 1
et Q est un radical organique formant un pont, lequel radical forme, avec les deux atomes de carbone voisins, un cycle benzole, pyridine, pyrrole ou furanne si n = 0, et un cycle pyrrolidine le cas échéant substitué tel que ci-dessus si n = 1,
ces cycles carbocycliques ou hétérocycliques, en tant qu'anneaux aromatiques, pouvant le cas échéant être complexés avec un métal de transition, et en présence d'une base.

**2.** Procédé selon la revendication 1, **caractérisé en ce que** $A^2$ est un azote et fait partie d'un cycle pyridine.

**3.** Procédé selon la revendication 2, **caractérisé en ce que** $R^1$ est un groupe de formule -OR, dans laquelle R a la signification désignée dans la revendication 1.

**4.** Procédé selon la revendication 1, **caractérisé en ce que** $A^1$ est un azote et fait partie d'un cycle pyridine.

**5.** Procédé selon la revendication 1, **caractérisé en ce que** $A^1$ et $A^5$ sont un azote et font partie d'un cycle pyrimidine.

**6.** Procédé selon la revendication 1, **caractérisé en ce que** $A^1$ et $A^4$ sont un azote et font partie d'un cycle pyrazine.

**7.** Procédé selon la revendication 1, **caractérisé en ce que** $A^1$, $A^3$ et $A^5$ sont un azote.

**8.** Procédé selon la revendication 4, 5, 6 ou 7 **caractérisé en ce que** $R^2$ est un groupe de formule -OR, dans laquelle R a la signification mentionnée dans la revendication 1.

**9.** Procédé selon la revendication 3 ou 8, **caractérisé en ce que** R est un groupe phényle le cas échéant substitué.

**10.** Procédé selon l'une des revendications 1 à 9, **caractérisé en ce que** $R^6$ est un hydrogène et $R^7$ est un groupe phényle le cas échéant substitué.

**11.** Procédé selon l'une quelconque des revendications 1 à 10 **caractérisé en ce que** X est un chlore.

**12.** Procédé selon l'une des revendications 1 à 11, **caractérisé en ce qu'**un ferrocène de formule générale

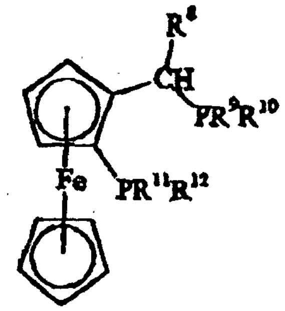

IVa,

dans laquelle $R^8$ est un hydrogène ou un méthyle et $R^9$ à $R^{12}$ ont la signification mentionnée dans la revendication 1, est utilisé en tant que diphosphine (IV).